# FASCICULE DE BREVET EUROPEEN

(11) **EP 1 941 913 B1**
(45) Date de publication et mention de la délivrance du brevet: **09.11.2011**
(21) Numéro de dépôt: 07291539.0
(22) Date de dépôt: 17.12.2007
(51) Int. Cl.: A61L 2/04, A61L 2/20, B29C 49/42, B29C 49/64, B65B 55/06, B65B 55/10

(54) **Procédé et dispositif de stérilisation de préformes**
Verfahren und Vorrichtung zur Sterilisierung von Vorformen
Method and device for sterilising preforms

(30) Priorité: 20.12.2006 FR 0611145
(43) Date de publication de la demande: 09.07.2008
(73) Titulaire: Sidel Participations, 76930 Octeville Sur Mer (FR)
(72) Inventeur: Quetel, François, c/o Sidel Participations, 76930 Octeville sur Mer (FR); Hebert, Stéphane, c/o Sidel Participations, 76930 Octeville sur Mer (FR)
(74) Mandataire: Habasque, Etienne J. Jean-François

(56) Documents cités:
- EP-A2- 0 243 003
- FR-A1- 2 766 121
- FR-A1- 2 774 912
- FR-A1- 2 789 932
- US-A1- 2004 191 114
- US-B1- 6 183 691

## Description

L'invention concerne un procédé et un dispositif de stérilisation de préformes.

L'invention concerne plus particulièrement un procédé de stérilisation d'une préforme en matière plastique destinée à être moulée, notamment par soufflage.

Le document WO 99/03667 A1 décrit une installation pour produire des bouteilles stériles à partir de préformes en matière plastique, du type dans lequel les préformes sont convoyées à l'intérieur de l'installation selon un flot continu qui circule de l'amont vers l'aval. L'installation met en oeuvre le procédé consistant à :
- mouiller la préforme en amont des moyens de chauffage,
- transférer la préforme vers les moyens de chauffage,
- chauffer par rayonnement la préforme mouillée afin de la stériliser.

Ce type d'installation présente l'inconvénient de nécessiter un débit de produit stérilisant et/ou une pression d'injection du produit stérilisant de valeurs importantes pour réussir à couvrir complètement les parois internes des préformes de manière à stériliser complètement l'intérieur des préformes.

Par conséquent, la consommation de l'installation en produit stérilisant est importante et l'opération de stérilisation coûteuse.

De plus, l'utilisation d'un débit important de produit stérilisant peut conduire au dépôt de gouttelettes (résiduelles) de produit stérilisant de taille important sur les parois internes des préformes. Or, lors du chauffage des préformes, ces gouttelettes produisent un effet loupe sur les rayonnements thermiques de chauffage, ce qui conduit à l'apparition de taches sur les parois des bouteilles issues des préformes concernées.

En effet, dans les installations de l'état de la technique, le produit stérilisant est pulvérisé sous la forme d'un brouillard et avec une pression obtenue en comprimant à des pressions d'environ 2 à 3 bars un gaz, tel que de l'air comprimé, qui est stérilisé et chauffé, par exemple à une température de l'ordre de 130°C, de manière à activer thermiquement le produit stérilisant.

C'est la raison pour laquelle les gouttelettes de produit stérilisant forment un excédent qui n'est pas entièrement vaporisé lors du chauffage, de telle sorte que chaque gouttelette provoque un effet loupe sur le matériau de la préforme, généralement en polyéthylène téréphtalate (PET), par le produit stérilisant.

Ce phénomène conduit à l'apparition de taches sur les parois des bouteilles, ce défaut d'aspect étant encore parfois appelé "peau d'orange".

De plus, les parois de la préformes ne sont pas uniformément recouvertes par les gouttelettes de produit stérilisant, de sorte que des zones non stérilisées subsistent entre chacune des gouttelettes, sur la surface des parois interne et/ou externe de la préforme.

De surcroît, suivant le profil de la paroi interne de chaque préforme, il n'est pas toujours possible d'atteindre à coup sûr le fond des préformes, même avec un débit et/ou une pression de valeurs importantes, en raison de la création d'un bouchon de surpression au fond des préformes.

Le document FR 2 774 912 A1 décrit un procédé de stérilisation d'une file de préformes par projection d'un flux de vapeur stérilisante dans une chambre de protection en surpression.

La présente invention vise donc à remédier aux inconvénients précédents et à proposer notamment un procédé et un dispositif de stérilisation qui ne conduise pas à l'apparition de gouttelettes tout en réduisant la consommation de produit stérilisant.

Dans ce but, l'invention propose un procédé de stérilisation d'une file de préformes en matière plastique destinées à être moulées, notamment par soufflage, comportant au moins les étapes suivantes :
- projeter un flux de vapeur stérilisante comportant un produit stérilisant vaporisé vers les préformes à stériliser, de manière à recouvrir au moins une paroi interne des préformes à stériliser avec ce produit, et
- chauffer par rayonnement les préformes recouvertes de produit stérilisant pour les porter à une température supérieure ou égale à une température d'activation du produit,
le procédé étant caractérisé en ce que
- la projection du flux de vapeur stérilisante est réalisée dans une chambre de protection,
- le chauffage est réalisé hors de la chambre de protection, et
- le flux de vapeur stérilisante est sous forme d'un jet de vapeur vaporisé sur les préformes, de manière à provoquer le dépôt par condensation d'un film de buée de produit stérilisant, sensiblement uniforme, sur au moins la paroi interne des préformes à stériliser.

D'autres caractéristiques du procédé sont décrites dans les revendications 2 à 7

L'invention a également pour objet un dispositif de stérilisation d'une file de préformes en matière plastique destinées à être moulées, notamment par soufflage suivant la revendication 8.

D'autres caractéristiques du dispositif sont décrites dans les revendications 9 à 11.

D'autres caractéristiques et avantages de l'invention apparaîtront à la lecture de la description détaillée qui suit pour la compréhension de laquelle on se reportera aux dessins annexés dans lesquels :
- la figure 1 est un schéma qui représente un exemple de réalisation d'une installation produisant des bouteilles stériles par soufflage et comportant une unité de stérilisation mettant en oeuvre le procédé de stérilisation selon l'invention ;
- la figure 2 est une vue en coupe axiale suivant le plan de coupe II-II de la figure 3, qui représente schématiquement une préforme dans l'unité de stérilisation de l'installation de la figure 1 ;
- la figure 3 est une vue de dessus qui représente une série de préformes au poste de projection de l'unité de stérilisation.

Dans la suite de la description, des éléments similaires ou identiques seront désignés par les mêmes références.

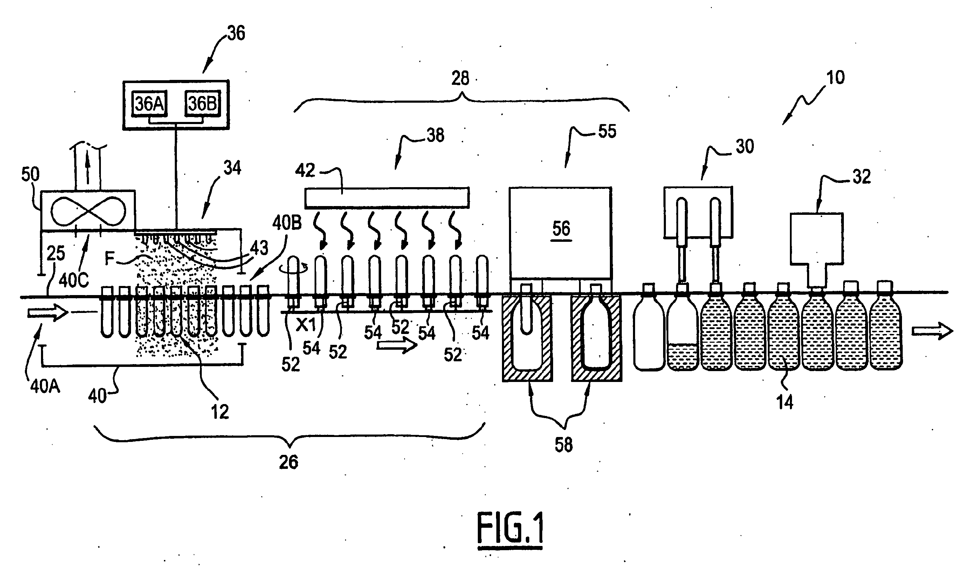

Sur la figure 1, on a représenté une installation 10 produisant des récipients tels que des bouteilles 14, en particulier des bouteilles stériles ou aseptisées, qui sont avantageusement obtenues par soufflage à partir de préformes 12 en matière plastique, par exemple en polyéthylène téréphtalate (PET).

Chaque préforme 12 a globalement la forme d'un tube à section longitudinale en U qui est fermé à une extrémité et dont l'autre extrémité possède déjà la forme définitive du col 16 de la bouteille 14.

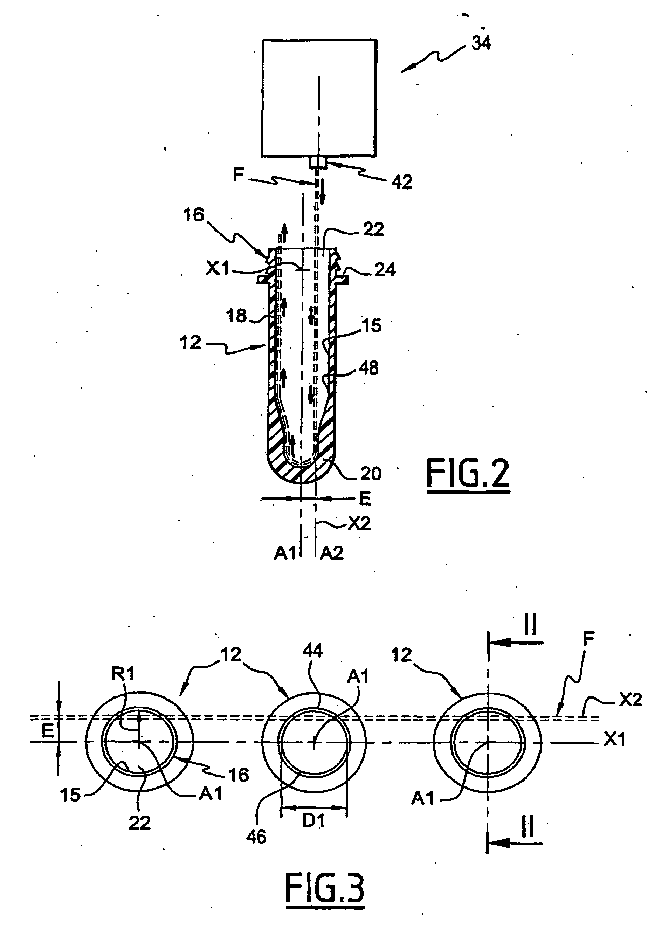

Sur la figure 2, une préforme 12 est représentée, à titre non limitatif, avec l'axe A1 de son corps 18 cylindrique qui s'étend verticalement et qui est confondu avec l'axe du col 16.

L'extrémité inférieure 20 de la préforme 12 est fermée, en forme générale de demi-sphère, tandis que son extrémité supérieure forme le col 16, qui délimite une ouverture intérieure 22 et qui est ici pourvu d'une collerette 24 radiale externe.

Les préformes 12 sont généralement réalisées selon un procédé de moulage par injection et moulées sur un autre lieu que celui où se trouve l'installation 10.

Pour certaines applications, les bouteilles 14 obtenues à partir des préformes 12 doivent présenter un certain degré de stérilité. C'est la raison pour laquelle on procède à une opération de stérilisation des préformes 12 dans l'installation 10 de production des bouteilles 14.

Plus précisément, l'opération de stérilisation concerne en priorité le col 16 et la paroi 15 interne de la préforme 12 correspondant à la paroi interne, qui délimite le volume intérieur de la bouteille 14 destiné à être rempli.

En référence à la Figure 1, l'installation 10 comprend des moyens d'acheminement des préformes sous la forme d'un rail de convoyage 25. Ainsi, les préformes 12 sont convoyées à l'intérieur de l'installation 10 selon un flot continu qui circule de l'amont vers l'aval, c'est-à-dire de la gauche vers la droite sur la figure 1.

L'installation 10 comporte, de l'amont vers l'aval, d'une part, une unité de stérilisation 26 des préformes 12 et, d'autre part, une unité de mise en forme 28 des préformes 12 stérilisées pour former les bouteilles 14.

Avantageusement, l'installation 10 comporte aussi, à la suite de l'unité de mise en forme 28, une unité de remplissage 30 et une unité de bouchage 32. Ces deux dernières unités sont bien connues et ne seront pas décrites plus précisément.

L'unité de stérilisation 26 comporte un poste de projection 34 de vapeur stérilisante, alimenté en vapeur stérilisante par des moyens 36 de préparation de vapeur stérilisante. L'unité de stérilisation 26 comporte également un poste de chauffage 38.

Les moyens 36 de préparation de vapeur stérilisante comportent des moyens de chauffage 36A d'un produit stérilisant, pour le vaporiser, et une source d'air 36B, avantageusement comprimé et/ou stérilisé par tout moyen approprié, qui est prévue pour propulser le produit stérilisant vaporisé à travers les buses décrites par la suite. Le mélange de produit stérilisant vaporisé et d'air forme le flux de vapeur stérilisante.

De préférence, l'air comprimé est déshydraté et circule à faible vitesse selon un écoulement directif de manière à constituer un vecteur pour le produit stérilisant vaporisé.

De préférence, le produit stérilisant est constitué d'un composé contenant du peroxyde d'hydrogène ou de peroxyde d'hydrogène vaporisé (H202) qui, au poste de projection 34, est projeté vers les préformes 12 sous la forme d'un jet de gaz comportant du produit stérilisant à l'état de vapeur, avantageusement un jet de vapeur sèche.

Par exemple, le produit stérilisant est un mélange de 25% d'H2O2 dans 75% d'eau.

La vapeur stérilisante est ainsi constituée, d'une part, du produit stérilisant vaporisé et, d'autre part, de l'air chaud.

De préférence, la proportion P (en volume) de produit stérilisant vaporisé par rapport à l'air chaud est comprise entre 10% et 15%. La proportion d'air chaud est donc de 100% - P.

Le poste de projection 34 comprend par ailleurs une chambre 40 de protection contre la diffusion de vapeurs.

La protection est destinée tout d'abord aux opérateurs de la machine, car les vapeurs de produit stérilisant sont généralement nocives pour la santé. La protection est en outre destinée aux autres éléments de l'installation 10, notamment le poste de chauffage 38, qui pourraient être dégradés par l'effet corrosif du produit stérilisant.

La chambre de protection 40 est hermétiquement fermée, sauf pour des ouvertures d'entrée 40A et de sortie 40B des préformes 12, et une ouverture supérieure d'extraction 40C des vapeurs. Les ouvertures 40A, 40B d'entrée/sortie des préformes sont de préférence juste conformées pour donner libre passage aux préformes 12. Des caches amovibles (non représentés) sont prévus pour obturer des portions des ouvertures 40A, 40B lorsque les préformes sont de petite dimension.

Le poste de projection 34 de l'unité de stérilisation 26 est pourvu d'au moins une buse 43 située dans la chambre de protection 40. Chaque buse 43 projette, en cours de traitement, un flux F de vapeur stérilisante sous la forme d'un jet de vapeur, ici vers le col 16 des préformes 12 à stériliser, de manière à provoquer le dépôt par condensation d'un film de buée de produit stérilisant, sensiblement uniforme, sur au moins la paroi interne 15 de la préforme, de préférence sur la paroi interne 15 et l'extérieur du col 16. Pour ce faire, le dispositif délivre un flux de vapeur qui vient lécher la paroi externe du col.

Selon le mode de réalisation représenté ici, notamment sur la figure 3, les préformes 12 défilent dans le poste de projection 34 de l'unité stérilisation 26 en étant alignées, en position verticale, suivant une direction horizontale longitudinale, dite direction de défilement X1, le col 16 vers le haut.

La direction de défilement X1 passe par les axes A1 des préformes 12 en cours de traitement.

Avantageusement, l'axe moyen de projection A2 des buses 43 est globalement parallèle à l'axe A1 de chaque préforme 12 en cours de traitement et cet axe A2 est excentré radialement, par rapport à l'axe A1 de la préforme 12, d'une valeur de décalage E déterminée.

De préférence, l'axe moyen de projection A2, qui est ici vertical, est excentré suivant un rayon intérieur R1 du col 16 qui est orthogonal à la direction de défilement X1.

Ainsi, la forme de chaque buse 43 permet de projeter, vers le bas, un flux F de vapeur stérilisante globalement sous forme de flux laminaire, c'est-à-dire sous la forme d'un rideau vertical longitudinal. A cet effet, chaque buse 43 comporte par exemple une fente ou un trou globalement circulaire de projection du flux F.

Le flux F laminaire s'étend ici globalement suivant un rideau contenu dans un plan vertical longitudinal, dit plan de projection X2, qui est décalé radialement, par rapport à la direction de défilement X1, d'une distance égale au décalage E.

De préférence, la valeur de décalage E est comprise entre une valeur minimale Emin sensiblement égale à 19% du diamètre intérieur D1 du col 16 de chaque préforme 12, et une valeur maximale Emax sensiblement égale à 32% de ce diamètre intérieur D1.

Selon un mode de réalisation avantageux, la valeur de décalage E est choisie fixe et sensiblement égale à 8 millimètres, de sorte qu'elle convient à des modèles de préformes 12 possédant des diamètres intérieurs D1 compris environ entre 25 et 42 millimètres.

Grâce à un tel agencement des buses 43, le flux F de vapeur stérilisante affleure sensiblement un premier secteur 44 de la paroi 15 interne de chaque préforme 12, de sorte que le flux F de vapeur stérilisante lèche ledit secteur 44.

En arrivant à l'extrémité inférieure 20 de la préforme 12, le flux F de vapeur stérilisante glisse sur le fond sensiblement hémisphérique de la préforme 12 et remonte le long d'un second secteur 46 de paroi 15 interne, diamétralement opposé au premier 44.

Ainsi, le flux F de vapeur stérilisante balaye globalement l'ensemble de la paroi 15 interne de chaque préforme 12, par écoulement de type laminaire.

Un tel agencement permet notamment d'empêcher la création d'un bouchon de surpression, dans le fond des préformes 12, qui empêcherait le produit stérilisant d'atteindre le fond.

Les buses 43 suivent la trajectoire des préformes et diffusent de la vapeur pendant un temps donné qui correspond au temps de dépôt.

L'état gazeux du flux F permet une diffusion uniforme sur toute la surface.

En particulier, la vitesse de propulsion de la vapeur stérilisante, à la sortie de la buse 43, est suffisamment faible pour permettre l'écoulement sensiblement de type laminaire.

Les préformes 12 parcourant l'installation 10, notamment l'unité de stérilisation 26, sont ici orientées verticalement avec le col 16 vers le haut, c'est-à-dire en position dite "col en haut".

Ainsi, au poste de projection 34, le flux F de vapeur stérilisante est vaporisé sur chaque préforme 12. La température T1 des préformes est inférieure à la température de condensation Tc du produit stérilisant, de manière qu'un film de buée 48 de produit stérilisant contenu dans le flux de vapeur stérilisante, se dépose uniformément par condensation au moins sur la paroi interne 15 et de préférence également sur l'extérieur du col 16 de la préforme 12 à stériliser.

A la sortie de la buse 43, la vapeur contenant le produit stérilisant vaporisé est à une température donnée sensiblement supérieure à la température d'évaporation Te du produit stérilisant, de sorte que le produit stérilisant se condense instantanément sur la préforme 12.

Dans le cas d'utilisation d'un mélange d'eau et de peroxyde d'hydrogène (H202), la température en sortie de buse est avantageusement supérieure à 106°C, de préférence comprise entre 110°C et 120°C.

Lorsque cette vapeur entre en contact avec chaque préforme 12, qui est plus froide, le produit stérilisant vaporisé se condense de manière que l'ensemble de la préforme 12, en particulier sur la paroi 15 interne, s'embue d'un film de buée 48 de produit stérilisant.

La buse 43 est ici réalisée de manière que le film de buée 48 sensiblement uniforme se dépose principalement sur le col 16 et sur l'ensemble de la surface de la paroi interne 15.

Avantageusement, le dépôt par condensation suivant le film de buée 48 uniforme de produit stérile permet, par rapport à l'état de la technique, d'éliminer sensiblement tout risque d'apparition de taches et d'aspect "peau d'orange".

Le poste de projection 34 comporte des moyens 50 d'extraction forcée de l'atmosphère de la chambre 40, par l'ouverture d'extraction 40C. Les moyens 50 ont pour but de récupérer le produit stérilisant vaporisé en excès, c'est-à-dire qui ne s'est pas déposé sur les préformes, ainsi que l'air chaud transportant le produit stérilisant. Les moyens d'extraction 50 permettent ainsi d'éviter sensiblement toute sortie de produit stérilisant vaporisé par les ouvertures d'entrée/sortie des préformes 40A, 40B.

Afin de ne pas perturber le fonctionnement de l'unité de stérilisation, les moyens d'extraction 50 sont réglés de manière que le débit d'extraction soit suffisamment faible pour que le flux de vapeur stérilisante ne soit pas dévié de manière sensible.

Les inventeurs ont remarqué que seulement 15% à 25% du débit de produit stérilisant vaporisé contenu dans le flux de vapeur stérilisante projeté par les buses 43 se déposait sur les préformes 12 pour former le film de buée 48. Ainsi, un réglage satisfaisant du débit d'extraction par l'ouverture d'extraction 40C est obtenu en réglant ce débit égal à la somme :
- d'un débit de fuite pénétrant par les ouvertures d'entrée/sortie 40A, 40B,
- de 75 % à 85% du débit de projection de produit stérilisant vaporisé.
- du débit d'air chaud (100% - P).

En d'autres termes, le débit d'extraction est réglé égal à la somme, d'une part, du débit de fuite pénétrant par les ouvertures d'entrée/sortie 40A, 40B et, d'autre part, du débit du flux de vapeur stérilisante moins 15% à 25% du débit de produit stérilisant projeté dans le flux de vapeur stérilisante.

En pratique, ce réglage satisfaisant est obtenu empiriquement, par l'observation de préformes de test en sortie du poste de projection 34, pour vérifier qu'un film de buée 48 adéquat est bien présent et qu'il n'y a pas d'apparition de condensat sur les parois internes de l'enceintes 40.

Le poste de chauffage 38 est situé hors de la chambre de protection 40, en aval de celle-ci. Les moyens de convoyage 25 procèdent, avant l'entrée au poste de chauffage 38, au retournement des préformes 12 qui défilent alors "col en bas". En effet, à cause de phénomènes physiques et d'effets dus à la ventilation, le flux de chaleur dans les fours est dirigé du bas vers le haut., Or, le col est la partie définitivement moulée dans le processus de soufflage et ne doit subir ni déformation, ni surchauffe. Le risque premier lié à une surchauffe est l'éclatement du col lors du moulage de la bouteille. Le col en position basse (préformes « col en bas ») est donc positionné de façon à éviter la surchauffe et est aussi protégé par des rampes refroidies (non représentées) du rayonnement IR.

Le poste de chauffage 38 comporte au moins un four 42 destiné à chauffer par rayonnement les préformes 12 munies du film de buée 48 pour les porter à une température T supérieure ou égale à une température Ta d'activation du produit stérilisant, de manière à stériliser au moins la paroi 15 interne des préformes 12, et de préférence également le col 16. Afin de ne pas surcharger la figure 1, le four 42 est disposé au-dessus des préformes 12, en vis-à-vis de leur fond 20, alors qu'en réalité les moyens chauffant constituant le four sont disposés de part et d'autre du cheminement des préformes dans le four.

Le poste de chauffage 38 a, en plus de la fonction d'activation du film de produit stérilisant, une fonction de préparation des préformes 12 au moulage.

Ainsi, les préformes 12 sont en fait chauffées jusqu'à une température de moulage Tm qui, selon le type de préforme, varie entre 95°C et 135°C. Cette température Tm est ici supérieure à la température d'activation Ta du produit stérilisant et à la température d'évaporation Te permettant l'élimination par évaporation du produit stérilisant, sans requérir de moyens supplémentaires.

En effet, la température d'activation Ta du peroxyde d'hydrogène (H202) est d'environ 70°C, c'est-à-dire ici une température inférieure à la température de moulage Tm.

De manière générale, le four 42 est configuré pour porter les préformes 12 munies du film de produit stérilisant, à la plus élevée des trois températures Tm, Ta et Te.

Le four 42 est de préférence muni d'orifices d'aération (non représentés) pour permettre le passage d'air soufflé afin de favoriser un chauffage homogène dans toute l'épaisseur de la préforme 12 sans surchauffer la couche de matière superficielle.

En effet, l'air soufflé permet d'évacuer la chaleur de convection provoquée par les moyens de chauffage pour favoriser la pénétration du rayonnement qu'ils produisent dans l'épaisseur de la matière constituant la préforme 12.

Pour plus de détails sur de tels fours 42 de chauffage de préformes, on se reportera par exemple aux documents suivants : EP-A-0.620.099 ou EP-A-0.564.354.

De préférence, le four 42 comporte des moyens de protection, notamment pour limiter la corrosion des parties ou pièces exposées au produit stérile qui s'évapore des préformes 12.

Les moyens d'acheminement 25 comportent avantageusement des moyens 52 pour mettre les préformes 12 en rotation sur elles-mêmes pendant leur circulation dans le four 42 de manière à assurer le chauffage en profondeur de la préforme 12, c'est-à-dire tant de l'extrémité inférieure 20 formant fond que du corps 18 cylindrique. Le document WO-A-00/48819, auquel on se reportera pour plus de détails, décrit un exemple de tels moyens.

Les moyens d'acheminement 25 comportent, en outre, au niveau du poste de chauffage 38, au moins un moyen, tel qu'un noyau interne 54, aussi appelé nez de tournette, qui pénètre axialement à l'intérieur du col 16 de chaque préforme 12 en obstruant tout ou partie de l'ouverture intérieure 22 délimitée par le col 16 de manière à accroître le degré de stérilisation par augmentation de la durée d'exposition de la paroi 15 interne de la préforme 12 au produit stérilisant.

Ainsi qu'on l'aura compris, on obtient donc, à la sortie du four 42, des préformes 12 qui sont principalement stériles à l'intérieur et qui doivent avantageusement rester stériles jusqu'à l'opération finale de bouchage.

L'unité de mise en forme 28 comprend, en plus du poste de chauffage 38, un poste de moulage 55. Ce poste de moulage 55 comporte un dispositif de soufflage 56 qui soumet chaque préforme 12 à une surpression interne de manière qu'elle prenne la forme de l'empreinte d'un moule 58, ce qui produit une bouteille 14 stérile.

Le poste de moulage 34 peut aussi comporter des moyens d'élongation (non représentés) qui étirent la préforme 12 vers le fond du moule 58 pendant l'opération de moulage.

On va à présent décrire le fonctionnement de l'unité de stérilisation 26.

Pour stériliser notamment la paroi 15 interne de la préforme 12, on procède successivement aux étapes suivantes :
- vérifier que la préforme 12 est à une température T1 inférieure à la température de condensation Tc du produit stérilisant ;
- amener la préforme dans la chambre 40,
- projeter un flux F de vapeur stérilisante, contenant un produit stérilisant vaporisé, sous forme d'un jet de vapeur vers le col 16 de la préforme 12 de manière à provoquer le dépôt par condensation d'un film de buée 48 de produit stérilisant sensiblement uniforme au moins sur la paroi 15 interne de la préforme 12 à stériliser ; et
- sortir la préforme munie du film de buée 48 hors de la chambre 40 chauffer par rayonnement la préforme 12 ainsi traitée afin de porter la préforme 12 à une température T2 supérieure ou égale à la température d'activation Ta du produit stérilisant, de manière à stériliser au moins la paroi 15 interne de la préforme 12.

La température T2 est aussi supérieure à la température d'évaporation Te du produit stérilisant, de manière à provoquer son élimination par évaporation simultanément à son activation thermique par rayonnement.

La température de condensation Tc du peroxyde d'hydrogène est supérieure à approximativement 35°C. Ainsi, lorsque la préforme 12 est à une température sensiblement égale à la température ambiante de l'installation 10, par exemple comprise entre 7°C et 35°C, on obtient facilement une bonne condensation du produit stérilisant vaporisé.

De plus, la vérification de température s'en trouve alors simplifiée. En effet, il n'est pas nécessaire de modifier la température de la préforme 12 par chauffage ou refroidissement, pour obtenir une condensation du produit stérilisant sur la paroi 15 interne de la préforme.

Selon une variante de réalisation (non représentée) de l'installation 10, une enceinte de confinement stérile peut être prévue pour permettre de contrôler et préserver la stérilité des préformes 12 stérilisées et des bouteilles 14 entre les différentes unités ou différents postes de l'installation.

Grâce à une installation 10 selon la figure 1, on obtient une réduction logarithmique du nombre de germes de l'ordre de 3D, ou encore 3 Log équivalent à 1000 unités (103).

De manière connue, la quantité de germes est par exemple dénombrée par comptage après des opérations de lavage, de filtration et de mise en culture.

On note que l'installation 10 a été représentée avec des unités de traitement telles que l'unité de stérilisation 26, l'unité de mise en forme 28, l'unité de remplissage 30, l'unité de bouchage 32. Ces unités sont alignées, à titre d'illustration, mais ces unités peuvent être agencées selon une configuration différente, notamment avec des moyens d'acheminement tournants tels que des carrousels.

## Revendications

1. Procédé de stérilisation d'une file de préformes (12) en matière plastique destinées à être moulées, notamment par soufflage, comportant au moins les étapes suivantes :
- projeter un flux de vapeur stérilisante comportant un produit stérilisant vaporisé vers les préformes à stériliser, de manière à recouvrir au moins une paroi interne (15) des préformes à stériliser avec ce produit, et
- chauffer par rayonnement les préformes recouvertes de produit stérilisant pour les porter à une température (T2) supérieure ou égale à une température d'activation (Ta) du produit,
procédé dans lequel :
la projection du flux de vapeur stérilisante est réalisée dans une chambre de protection (40),
- le chauffage est réalisé hors de la chambre de protection (40), et
- le flux de vapeur stérilisante est sous forme d'un jet de vapeur (F) vaporisé sur les préformes (12), de manière à provoquer le dépôt par condensation d'un film de buée (48) de produit stérilisant, sensiblement uniforme, sur au moins la paroi interne (15) des préformes à stériliser,
le procédé étant **caractérisé en ce qu'**il comporte une étape d'extraction de l'atmosphère de la chambre de protection (40), afin d'éviter sensiblement toute sortie de produit stérilisant vaporisé, par des ouvertures d'entrée/sortie (40A, 40B) des préformes, ménagées dans la chambre de protection.

2. Procédé de stérilisation selon la revendication 1, **caractérisé en ce que** le jet de vapeur (F) s'étend suivant un rideau, et **en ce que** les préformes (12) défilent dans ce rideau.

3. Procédé de stérilisation selon la revendication 1 ou 2, **caractérisé en ce que** l'extraction est réalisée à un débit d'extraction suffisamment faible pour que le flux de produit stérilisant ne soit pas dévié de manière sensible.

4. Procédé de stérilisation selon la revendication 3, **caractérisé en ce que** la chambre de protection (40) n'est percée que d'ouvertures d'entrée/sortie (40A, 40B) de la préforme (12) et d'une ouverture d'extraction (40C).

5. Procédé de stérilisation selon la revendication 4, **caractérisé en ce que** le débit d'extraction par l'ouverture d'extraction (40C) est réglé égal à la somme, d'une part, d'un débit de fuite pénétrant par les ouvertures d'entrée/sortie (40A, 40B) et, d'autre part, du débit du flux de vapeur stérilisante moins 15% à 25% du débit de produit stérilisant projeté dans le flux de vapeur stérilisante.

6. Procédé selon l'une quelconque des revendications précédentes, **caractérisé en ce que** la température (T2) à laquelle les préformes (12) sont portées est sensiblement égale à une température de moulage (Tm) par soufflage des préformes (12) qui est respectivement supérieure à la température d'activation (Ta) et à une température d'évaporation (Te) du produit stérilisant.

7. Procédé de stérilisation selon l'une quelconque des revendications 1 a 6, **caractérisé en ce que** lors de ladite projection, les préformes (12) sont à une température (T1) sensiblement égale à la température ambiante de l'installation, par exemple comprise entre 7°C et 35°C.

8. Dispositif de stérilisation d'une file de préformes (12) en matière plastique destinées à être moulées, notamment par soufflage, comprenant :
- un poste de projection (34) comportant au moins une buse (43) pour projeter un flux de vapeur stérilisante comportant un produit stérilisant vaporisé, vers les préformes (12) à stériliser, de manière à recouvrir au moins une paroi interne (15) des préformes à stériliser avec ce produit,
- un poste de conditionnement thermique (38) comportant un four (42) par rayonnement destiné à porter les préformes à une température (T2) supérieure ou égale à une température d'activation (Ta) du produit stérilisant de manière à stériliser au moins la paroi interne (15) des préformes,
- des moyens d'acheminement (25) des préformes au poste de projection, puis au poste de conditionnement thermique,
dispositif dans lequel:
- le poste de projection (34) comporte en outre une chambre de protection (40) dans laquelle est située chaque buse (43).
- le poste de conditionnement thermique (38) est situé hors de la chambre de projection (40), et
- au poste de projection (34), le flux de vapeur stérilisante est vaporisé sous forme d'un jet de vapeur (F) vaporisée sur les préformes (12) de manière à provoquer le dépôt par condensation d'un film de buée (48) de produit stérilisant, sensiblement uniforme, sur au moins la paroi interne (15) des préformes à stériliser,
le dispositif étant **caractérisé en ce que** le poste de projection (34) comporte des moyens d'extraction (50) de l'atmosphère de la chambre de protection (40) afin d'éviter sensiblement toute sortie de produit stérilisant vaporisé, par des ouvertures d'entrée/surtie (40A. 40B) des préformes ménagées dans la chambre de protection.

9. Dispositif de stérilisation selon la revendication 8, **caractérisé en ce que** les moyens d'extraction (50) sont réglés de manière que le débit d'extraction soit suffisamment faible pour que le flux de produit stérilisant ne soit pas dévié de manière sensible.

10. Dispositif de stérilisation selon l'une quelconque des revendications 8 ou 9, **caractérisé en ce que** chaque buse (43) possède un axe moyen de projection (A2) de la vapeur stérilisante vers un col (16) de la préforme, cet axe moyen de projection (A2) étant, d'une part, globalement parallèle à l'axe (A1) des préformes (12) acheminées, et, d'autre part excentré radialement par rapport à l'axe (A1) des préformes (12).

11. Dispositif de stérilisation selon l'une quelconque des revendications 8 à 10, **caractérisé en ce que** les moyens d'acheminement (25) comportent au moins un moyen (54), tel qu'un noyau interne, qui pénètre axialement à l'intérieur d'un col (16) de chaque préforme (12) en obstruant tout ou partie de l'ouverture intérieure (22) délimitée par le col (16), de manière à accroître le degré de stérilisation par augmentation de la durée d'exposition de la paroi (15) interne de la préforme (12) avec le produit stérilisant.

## Claims

1. A method for sterilizing a line of preforms (12) made of plastics material that are intended to be moulded, especially by blow moulding, including at least the following steps:
- spraying a flow of sterilizing vapour, including a vaporized sterilizing product, towards the preforms to be sterilized, so as to cover at least an inner wall (15) of the preforms to be sterilized with the product, and
- heating, by radiation, the preforms covered with sterilizing product to bring them to a temperature (T2) at or above an activation temperature (Ta) for the product,
in which method:
- the spraying of the flow of sterilizing vapour is carried out in a protective chamber (40),
- heating is carried out outside the protective chamber (40), and
- the flow of sterilizing vapour is in the form of a jet of vapour (F) vaporised, onto the preforms (12), in such a way as to bring about the deposition, by condensation, of a substantially uniform film of condensate (48) of sterilizing product on at least the inner wall (15) of the preforms to be sterilized,
the method being **characterized in that** it includes a step of extraction of the atmosphere from the protective chamber (10), in order substantially to avoid any emergence of the vaporized sterilizing product through entry/exit openings (40A, 40B) for the preforms, provided in the protective chamber.

2. A sterilizing method according to claim 1, **characterized in that** the jet of vapour (F) extends in a curtain, and **in that** the preforms (12) file through in this curtain.

3. A sterilizing method according to claim 1 or 2, **characterized in that** extraction is carried out at an extraction rate low enough for the flow of sterilizing product not to be noticeably deflected.

4. A sterilizing method according to claim 3, **characterized in that** the protective chamber (40) is pierced only by entry/exit opening (40A, 40B) for the preform (12) and by an extraction opening (40C).

5. A sterilizing method according to claim 4, **characterized in that** the rate of extraction through the extraction opening (40C) is regulated to be equal to the sum, on the one hand, of an escape flow rate penetrating through the entry/exit openings (40A, 40B) and, on the other hand, of the flow rate of the flow of sterilizing vapour less 15% to 25% of the flow rate of sterilizing product sprayed in the flow of sterilizing vapour.

6. A method according to any one of the preceding claims, **characterized in that** the temperature (T2) to which the preforms (12) are brought is substantially equal to a moulding temperature (Tm) for blow moulding of the preforms (12) which is respectively above the activation temperature (Ta) and an evaporation temperature (Te) of the sterilizing product.

7. A sterilizing method according to any one of claims 1-6, **characterized in that**, during said spraying, the performs (12) are at a. temperature (T1) substantially equal to the ambient temperature of the plant, for example between 7°C and 35°C.

8. A device for sterilizing a line of preforms (12.) made of plastics material that are intended to be moulded, especially by blow moulding, comprising :
- a spraying station (34) including at least one nozzle (43) for spraying a flow of sterilizing vapour, including a vaporized sterilizing product, towards the preforms (12) to be sterilized, in such a way as to cover at least an inner wall (15) of the preforms to be sterilized with the product,
- a heat conditioning station (38) including a radiation oven (42) intended to bring the preforms to a temperature (T2) at or above an activation temperature (Ta) for the sterilizing product, in such a way as to sterilize at least the inner wall (15) of the preforms,
- transport means (25) for transporting the preforms to the spraying station, then to the heat conditioning station,
in which device:
- the spraying station (34) further includes a protective chamber (40) in which each nozzle (43) is located,
- the heat conditioning station (38) is located outside the protective chamber (40), and
- at the spraying station (34), the flow of sterilizing vapour is vaporized in the form of a jet of vapour (F) vaporized onto the preforms (12) in such a way an to bring about the deposition, by condensation, of a substantially uniform film of condensate (48) of sterilizing product on at least the inner wall (15) of the preforms to be sterilized,
the device being **characterized in that** the spraying station (34) includes extraction means (50) for extracting the atmosphere from the protective chamber (40), in order substantially to avoid any emergence of the vaporized sterilizing product through entry/exit openings (40A, 40B) for the preforms, provided in the protective chamber.

9. A sterilizing device according to claim 8, **characterized in that** the extraction means (50) are regulated in such a way that the extraction rate is low enough for the flow of sterilizing product not to be noticeably deflected.

10. A sterilizing device according to either claim 8 or claim 9, **characterized in that** each nozzle (43) has a mean spraying axis (A2) for spraying the sterilizing vapour towards a neck (16) of the preform, the mean spraying axis (A2) being on the one hand generally parallel to the axis (A1) of the transported preforms (12), and on the other hand radially eccentric relative to the axis (Al) of the preforms (12).

11. A sterilizing device according to any one of claims 8 to 10, **characterised in that** the transport means (25) include at least one means (54), such as an inner core, which penetrates axially inside a neck (16) of each preform (12), obstructing all or part of the inner opening (22) defined by the neck (16), so as to increase the degree of sterilization by increasing the length of time for which the inner wall (15) of the preform (12) is exposed to the sterilizing product.

## Patentansprüche

1. Verfahren zum Sterilisieren einer Reihe von Kunststoffvorformlingen (12), die insbesondere durch Blasen geformt werden sollen, das wenigstens die folgenden Schritte umfasst:
- Ausstoßen eines sterilisierenden Dampfstroms, der ein zerstäubtes Sterilisationsprodukt enthält, zu den zu sterilisisrenden Vorformlingen, derart, dass wenigstens eine Innenwand (15) der zu sterilisierenden Vorformlinge mit diesem Produkt bedeckt wird, und
- Erhitzen der mit dem sterilisierenden Produkt abgedeckten Vorformlinge durch Strahlung, um sie auf eine Temperatur (T2) größer oder gleich einer Aktivierungstemperatur (Ta) des Produkts zu bringen,
wobei in dem Verfahren:
- das Ausstoßen des sterilisierenden Dampfstroms in einer Schutzkammer (40) erfolgt,
- der sterilisierende Dampfstrom die Form eines auf die Vorformlinge (12) gerichteten zerstäubten Dampfstrahls hat, derart, dass der Niederschlag durch Kondensation eines Beschlagfilms (48) des sterilisierenden Produkts im Wesentlichen gleichmäßig wenigstens auf der Innenwand (15) der zu sterilisierenden Vorformlinge hervorgerufen wird,
wobei das Verfahren **dadurch gekennzeichnet ist, dass** es einen Schritt des Extrahierens der Atmosphäre aus der Schutzkammer (40) umfasst, um im Wesentlichen jeglichen Austritt des zerstäubten sterilisierenden Produkts durch die Einlass-/Auslassöffnungen (40A, 40B) für die Vorformlinge, die in der Schutzkammer ausgebildet sind, zu vermeiden.

2. Steritisationsverfahren nach Anspruch 1, **dadurch gekennzeichnet, dass** sich der Dampfstrom (F) längs eines Vorhangs erstreckt und dass sich die Vorformlinge (12) in diesem Vorhang vorbei bewegen.

3. Sterilisationsverfahreri nach Anspruch 1 oder 2, **dadurch gekennzeichnet, dass** das Extrahieren mit einem ausreichend geringen Extraktionsdurchsatz erfolg, damit der Strom des sterilisierenden Produkts nicht wesentlich abgelehnt wird.

4. Sterilisationsverfahren nach Anspruch 3, **dadurch gekennzeichnet, dass** die Schutzkammer (40) nur mit Einlass-/Auslassöffnungen (40A, 40B) für den Vorformling (12), und mit einer Extraktionsöffnung (40C) durchsetzt ist.

5. Sterüisationsverfaltrer; nach Anspruch 4, **dadurch gekennzeichnet, dass** der Extraktionsdurchsatz durch die Extraktionsöffnung (40C) so gesteuert wird, dass er gleich der Summe einerseits aus dem Leckdurchsatz, der durch die Einlass-/Auslassöffnungen (40A, 40B) dringt, und andererseits aus dem Durchsatz des sterilisierenden Dampfstroms abzüglich 15 % bis 25 % des Durchsatzes des sterilisierenden Produkts, das in dem sterilisierenden Dampfstrom ausgestoßen wird, ist.

6. Verfahren nach einem der vorhergehenden Ansprüche, **dadurch gekennzeichnet, dass** die Temperatur (T2), auf die die Vorformlinge (12) gebracht werden, im Wesentlichen gleich einer Temperatur (Tm) zum Gießen der Vorformlinge (12) durch Blasen, die höher ais die Aktivierungstemperatur (Ta) bzw. als die Verdampfungstemperiitur (Tc) des sterilisierenden Produkts ist, ist.

7. Steriiisattonsverfahren nach einem der Ansprüche 1 bis 6, **dadurch gekennzeichnet, dass** beim Ausstoßen die Vorformlinge (12) eine Temperatur (T1) haben, die im Wesentlichen gleich der Umgebungstemperatur der Anlage ist und beispielsweise im Bereich von 7 °C bis 35 °C liegt.

8. Vorrichtung zum Sterilisieren einer Reihe von Kunststoffvorforrlingen (12), die insbesondere durch Blasen geformt werden sollen, die umfasst:
- eine Ausstoßstation (34), die wenigstens eine Düse (43) umfasst, um einen sterilisierenden Dampfstrom, der ein zerstäubtes sterilisierendes Produkt enthält, zu den zu sterilisierenden Vorformlingen (12) auszustoßen, derart, dass wenigstens eine Innenwand (15) der zu sterilisierenden Vorformlinge mit diesem Produkt bedeckt wird,
- eine thermische Aufbereitungsstation (38), die einen Strahlungsofen (42) enthält, der die Vorformlinge auf eine Temperatur (T2) bringen soll, die größer oder gleich einer Aktivierungstemperatur (Ta) des zu sterilisierenden Produkts ist, derart, dass wenigstens die Innenwand (15) der Vorformlinge sterilisiert wird,
- Mittel (25) zum Führen der Vorformlinge zu der Ausstoßstation und dann zu der thermischen Aufbereitungsstation,
wobei in der Vorrichtung:
- die Ausstoßstation (34) außerdem eine Schutzkammer (40) aufweist, in der sich jede Düse (43) befindet,
- die thermische Aufbereitungsstation (38) sich außerhalb der Schutzkammer (40) befindet und
- in der Ausstoßstation (34) der sterilisierende Dampfstrom in Form eines auf die Vorformlinge (12) gerichteten zerstäubten Dampfstrahls (F) ausgestoßen wird, derart, das der Niederschlag durch Kondensation eines Beschlagfilms (48) aus dem sterilisierenden Produkt im Wesentlichen gleichmäßig wenigstens auf der Innenwand (15) der zu sterilisierenden Vorformlinge hervorgerufen wird,
wobei die Vorrichtung **dadurch gekennzeichnet ist, dass** die Ausstoß station (34) Mitte (50) zum Extrahieren der Atmosphäre aus der Schutzkammer (40) umfasst, um im Wesentlichen jeglichen Austritt des zerstäubten sterilisierenden Produkts durch die Einiass-/Attslassöffnungen (40A, 40B) für die Vorformlinge, die in der Schutzkammer ausgebildet sind, zu vermeiden.

9. Sterilisationsvorrichtung nach Anspruch 8, **dadurch gekennzeichnet, dass** die Extraktionstriittel (50) in der Weise gesteuert werden, das der Extraktionsdurchsatz ausreichend gering ist, damit der Strom des sterilisierenden Produkts nicht wesentlich abgelenkt wird.

10. Sterilisationsvorrichtung nach einem der Ansprüche 8 oder 9, **dadurch gekennzeichnet, dass** jede Düse (43) eine mittige Ausstoßachse (A2) für den sterilisierenden Dampf zu einem Hals (16) des Vorformlings besitzt, wobei diese mittige Ausstoßachse (A2) einerseits im allgemeinen zu der Achse (A1) der geführten Vorformlinge (72) parallel und andererseits in Bezug auf die Achse (A1) der Vorformlinge (12) radial exzentrisch ist.

11. Sterilisationsvorrichtung nach einem der Ansprüche 8 bis 10, **dadurch gekennzeichnet, dass** die Führungsmittel (25) wenigstens ein Mittel (54) wie etwa einen Innenkern aufweisen, der axial in einen Hals (16) jedes Vorformlings (12) eindringt und dabei die durch einen Hals (16) begrenzte Innenöffnung (22) ganz oder teilweise verschließt, derart, dass der Sterilsationsgrad durch Erhöhen der Dauer, während der die Innenwand (15) des Vorformlings (12) dem sterilisierenden Produkt ausgesetzt ist, erhöht wird.
